# EUROPEAN PATENT SPECIFICATION

(11) **EP 4 072 638 B1**
(45) Date of publication and mention of the grant of the patent: **12.06.2024**
(21) Application number: 20820836.3
(22) Date of filing: 04.12.2020
(51) Int. Cl.: A61M 16/06, A61M 16/08

(54) **MASK TO TUBING ADAPTOR FOR PATIENT INTERFACE DEVICE AND PATIENT INTERFACE DEVICE INCLUDING SAME**
MASKE FÜR SCHLAUCHADAPTER FÜR PATIENTENSCHNITTSTELLENVORRICHTUNG UND PATIENTENSCHNITTSTELLENVORRICHTUNG DAMIT
ADAPTATEUR DE MASQUE À TUBE POUR DISPOSITIF D'INTERFACE PATIENT ET DISPOSITIF D'INTERFACE PATIENT LE COMPRENANT

(30) Priority: 12.12.2019 US 201962946997 P
(43) Date of publication of application: 19.10.2022
(73) Proprietor: Koninklijke Philips N.V., 5656 AG Eindhoven (NL)
(72) Inventor: HAIBACH, Richard Thomas, 5656 AE Eindhoven (NL); SOFRANKO, Richard Andrew, 5656 AE Eindhoven (NL); COLDREN, Kevin Anthony, 5656 AE Eindhoven (NL)
(74) Representative: Philips Intellectual Property & Standards
(86) International application number: PCT/EP2020/084642
(87) International publication number: WO 2021/115955

(56) References cited:
- US-A1- 2005 199 242
- US-A1- 2017 049 984
- US-A1- 2018 099 113
- US-B2- 8 701 668
- US-B2- 10 092 720

## Description

### BACKGROUND OF THE INVENTION

### 1. Field of the Invention

The present invention pertains to patient interface devices for use in delivering a flow of a positive pressure breathing gas to an airway of a patient. More particularly, the present invention pertains to patient interface devices utilizing one or more adaptors to couple a tubing assembly and mask thereof. The present invention further pertains to methods for assembling patient interface devices utilizing such adaptors.

### 2. Description of the Related Art

Many individuals suffer from disordered breathing during sleep. Sleep apnea is a common example of such sleep disordered breathing suffered by millions of people throughout the world. One type of sleep apnea is obstructive sleep apnea (OSA), which is a condition in which sleep is repeatedly interrupted by an inability to breathe due to an obstruction of the airway; typically the upper airway or pharyngeal area. Obstruction of the airway is generally believed to be due, at least in part, to a general relaxation of the muscles which stabilize the upper airway segment, thereby allowing the tissues to collapse the airway. Another type of sleep apnea syndrome is a central apnea, which is a cessation of respiration due to the absence of respiratory signals from the brain's respiratory center. An apnea condition, whether obstructive, central, or mixed, which is a combination of obstructive and central, is defined as the complete or near cessation of breathing, for example a 90% or greater reduction in peak respiratory airflow.

Those afflicted with sleep apnea experience sleep fragmentation and complete or nearly complete cessation of ventilation intermittently during sleep with potentially severe degrees of oxyhemoglobin desaturation. These symptoms may be translated clinically into extreme daytime sleepiness, cardiac arrhythmias, pulmonary-artery hypertension, congestive heart failure and/or cognitive dysfunction. Other consequences of sleep apnea include right ventricular dysfunction, carbon dioxide retention during wakefulness, as well as during sleep, and continuous reduced arterial oxygen tension. Sleep apnea sufferers may be at risk for excessive mortality from these factors as well as by an elevated risk for accidents while driving and/or operating potentially dangerous equipment.

Even if a patient does not suffer from a complete or nearly complete obstruction of the airway, it is also known that adverse effects, such as arousals from sleep, can occur where there is only a partial obstruction of the airway. Partial obstruction of the airway typically results in shallow breathing referred to as a hypopnea. A hypopnea is typically defined as a 50% or greater reduction in the peak respiratory airflow. Other types of sleep disordered breathing include, without limitation, upper airway resistance syndrome (UARS) and vibration of the airway, such as vibration of the pharyngeal wall, commonly referred to as snoring.

It is well known to treat sleep disordered breathing by applying a continuous positive air pressure (CPAP) to the patient's airway. This positive pressure effectively "splints" the airway, thereby maintaining an open passage to the lungs. It is also known to provide a positive pressure therapy in which the pressure of gas delivered to the patient varies with the patient's breathing cycle, or varies with the patient's breathing effort, to increase the comfort to the patient. This pressure support technique is referred to as bi-level pressure support, in which the inspiratory positive airway pressure (IPAP) delivered to the patient is higher than the expiratory positive airway pressure (EPAP). It is further known to provide a positive pressure therapy in which the pressure is automatically adjusted based on the detected conditions of the patient, such as whether the patient is experiencing an apnea and/or hypopnea. This pressure support technique is referred to as an auto-titration type of pressure support, because the pressure support device seeks to provide a pressure to the patient that is only as high as necessary to treat the disordered breathing.

Pressure support therapies as just described involve the placement of a patient interface device including a mask component having a soft, flexible sealing cushion on the face of the patient. The mask component may be, without limitation, a nasal mask that covers the patient's nose, a nasal/oral mask that covers the patient's nose and mouth, or a full face mask that covers the patient's face. Such patient interface devices may also employ other patient contacting components, such as forehead supports, cheek pads and chin pads. The patient interface device is typically secured to the patient's head by a headgear component. The patient interface device is connected to a gas delivery tube or conduit and interfaces the pressure support device with the airway of the patient, so that a flow of breathing gas can be delivered from the pressure/flow generating device to the airway of the patient.

In order to ensure patient compliance with a prescribed therapy regiment, it is imperative that a patient interface device and components thereof provide a good seal, fit comfortably, and generally provide a good experience to the patient. In attempting to achieve all of these goals to patients of a myriad of different sizes and shapes, designers and manufacturers of patient interface devices commonly offer various designs and sizings of such designs from which prescribers and patients can attempt to find the best solution for the needs of a particular patient. While such approach generally provides a good variety of possible solutions for a given patient, there are still times where a best solution for a particular patient may be provided via a combination of patient interface components from different manufacturers. However, in many cases, components from one manufacturer are not made to readily interact with components from another manufacturer, thus making interchanging components from different manufacturers difficult, if not impossible.

US 2018/099113 A1 discloses a tubing assembly for use with a patient interface device and includes a manifold portion structured to be coupled to a conduit carrying a flow of breathing gas and a number of tubular portions. Each tubular portion comprises an adjustment element which provides for a characteristic of the tubular portion to be selectively varied.

US 10092720 B2 discloses an apparatus for delivering a flow of breathable gas to a patient and includes a nasal cannula, which is positioned on the face of the patient by a headgear.

US 8701668 B2 discloses a nasal assembly which includes a patient interface with a pair of nozzles structured to sealingly communicate with nasal passages of a patient's nose in use. Headgear is provided to maintain the patient interface in a desired position on the patient's face.

US 2017/0049984 A1 discloses a patient interface comprising a frame, a headgear, a manifold and two nasal prongs. The frame may be recessed from the face of the patient, and preferably from the manifold so that the manifold may deform or move with respect to the face of the patient.

US 2005/0199242 A1 discloses a patient interface device that includes a frame sized and configured to span at least a portion of a patient's face while remaining below the patient's eyes when the patient interface device is donned by the patient.

### SUMMARY OF THE INVENTION

The invention is defined by the claims. Embodiments of the present invention address shortcomings in the art by providing for components from different manufacturers to be readily coupled together. As one aspect of the invention, a respiratory interface system for use in delivering a flow of a positive pressure breathing gas to an airway of a patient is provided. The respiratory interface system comprises a patient interface device comprising: a tubing assembly structured to be disposed on the head of the patient; a mask having a sealing element structured to sealingly engage about the airway of the patient; and an adaptor, wherein the mask is coupled to the tubing assembly via the adaptor, and wherein the tubing assembly, the mask, and the adapter define a pathway structured to conduct the flow of the positive pressure breathing gas to the airway of the patient.

The tubing assembly may comprise: a manifold portion structured to be disposed at or about the top of the head of the patient; and a number of tubular portions, each extending from the manifold portion to a distal end that is coupled to the mask via the adaptor.

Each tubular portion may be sized and configured to extend down along a respective side of the head of the patient from the manifold portion and generally bend so as to curve along a respective cheek of the patient near the cheekbone of the patient.

The adaptor may comprise: a central flange portion that extends generally radially outward from a central aperture; a first hollow male connector extending from the central flange and coupled with a correspondingly-shaped female connector of the tubing assembly; and a second male connector extending from the central flange and coupled with a correspondingly-shaped female connector of the mask.

The first hollow male connector may comprise a first pair of prongs that extend outward from the flange portion in a first direction, and the second hollow male connector may comprise a second pair of prongs that extend outward from the flange portion in a second direction opposite the first direction.

Each prong of the first pair of prongs and the second pair of prongs may be arcuate shaped in a cross-section parallel to the central flange portion, and each prong may comprise a tapered portion at a leading end thereof that transitions to a raised portion positioned inward toward the flange portion from the tapered portion.

The adaptor may comprise: a body defining a passage therethrough that extends between a first opening and a second opening defined in an outer surface of the body, the first opening may define a female connector coupled with a correspondingly-shaped hollow male connector of the mask, and the second opening may define a female connector coupled with a correspondingly-shaped hollow male connector of the tubing assembly.

The hollow male connector of the mask may comprise a first pair of prongs that extend outward from an end of the mask, and the hollow male connector of the tubing assembly may comprise a second pair of prongs that extend outward from a distal end of a tubular portion of the tubing assembly.

Each prong of the first pair of prongs and the second pair of prongs may be arcuate shaped in a cross-section, and each prong may comprise a tapered portion at a leading end thereof that transitions to a raised portion positioned inward from the tapered portion.

The respiratory interface system may further comprise: a pressure generating device structured to produce the flow of the positive pressure breathing gas; and a delivery conduit fluidly coupled between the pressure generating device and the tubing assembly, the delivery conduit being structured to convey the flow of the positive pressure breathing gas from the pressure generating device to the tubing assembly.

As another aspect of the invention a method of assembling a patient interface device for use in providing a flow of a positive pressure breathing gas to an airway of a patient is provided. The patient interface device includes: a mask having a sealing element structured to sealingly engage about the airway of the patient, and a tubing assembly having a manifold portion and a pair of tubular portions, each tubular portion extending from the manifold portion to a distal end, wherein the tubing assembly is structured to convey the flow of the positive pressure breathing gas to the mask and the mask is further structured to convey the flow to the airway of the patient. The method comprises: connecting the distal end of a first one of the pair of tubular portions to the mask via a first adaptor; and connecting the distal end of a second one of the pair of tubular portions to the mask via a second adaptor.

Connecting the distal end of the first one of the pair of tubular portions to the mask via the first adaptor may comprise: inserting a first hollow male connector of the first adaptor into a female connector of the mask, and inserting a second hollow male connector of the first adaptor into a female connector of the first one of the pair of tubular portions; and connecting the distal end of the second one of the pair of tubular portions to the mask via a second adaptor may comprise: inserting a first hollow male connector of the second adaptor into a female connector of the mask, and inserting a second hollow male connector of the second adaptor into a female connector of the second one of the pair of tubular portions.

Connecting the distal end of the first one of the pair of tubular portions to the mask via the first adaptor may comprise: inserting a hollow male connector of the first one of the pair of tubular portions into a first female connector of the first adaptor, and inserting a first hollow male connector of the mask into a second female connector of the first adaptor; and connecting the distal end of the second one of the pair of tubular portions to the mask via a second adaptor may comprise: inserting a hollow male connector of the second one of the pair of tubular portions into a first female connector of the second adaptor, and inserting a second hollow male connector of the mask into a second female connector of the second adaptor.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 is a partially schematic depiction of a respiratory interface system for use in providing a flow of positive pressure breathing gas to the airway of a patient in accordance with one example embodiment of the present invention, shown with a patient interface device thereof disposed on the head of a patient;
FIG. 2 is a perspective view of an adaptor in accordance with one example embodiment of the present invention;
FIG. 3 is a perspective view of a patient interface device in accordance with one example embodiment of the present invention including two adaptors such as shown in FIG. 2;
FIG. 4 is an exploded view of a portion of the patient interface device of FIG. 3;
FIG. 5 is a perspective view of another adaptor in accordance with another example embodiment of the present invention;
FIG. 6 is an elevation sectional view of the adaptor of FIG. 5 taken along line A-A of FIG. 6;
FIG. 7 is a perspective view of a patient interface device in accordance with another example embodiment of the present invention including two adaptors such as shown in FIGS. 3 and 4; and
FIG. 8 is an exploded view of a portion of the patient interface device of FIG. 7.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

As used herein, the statement that two or more parts or components "engage" one another shall means that the parts exert a force against one another either directly or through one or more intermediate parts or components.

As used herein, the word "unitary" means a component is created as a single piece or unit. That is, a component that includes pieces that are created separately and then coupled together as a unit is not a "unitary" component or body.

As used herein, the term "number" shall mean one or an integer greater than one (i.e., a plurality).

As used herein, a "connector" is one element of a connector assembly. That is, a connector assembly includes at least two components, or connector components, that are structured to be coupled together. It is understood that the elements of a connector assembly are compatible with each other. For example, in a connector assembly, if one coupling element is a snap socket, the other connector element is a snap plug.

As used herein, "correspond" indicates that two structural components are sized and shaped to be similar to each other and may be coupled with a minimum amount of friction. Thus, an opening which "corresponds" to a member is sized slightly larger than the member so that the member may pass through the opening with a minimum amount of friction. This definition is modified if the two components are said to fit "snugly" together or "snuggly correspond." In that situation, the difference between the size of the components is even smaller whereby the amount of friction increases. If the element defining the opening and/or the component inserted into the opening is/are made from a deformable or compressible material, the opening may even be slightly smaller than the component being inserted into the opening. This definition is further modified if the two components are said to "substantially correspond." "Substantially correspond" means that the size of the opening is very close to the size of the element inserted therein. That is, not so close as to cause substantial friction, as with a snug fit, but with more contact and friction than a "corresponding fit," i.e. a "slightly larger" fit.

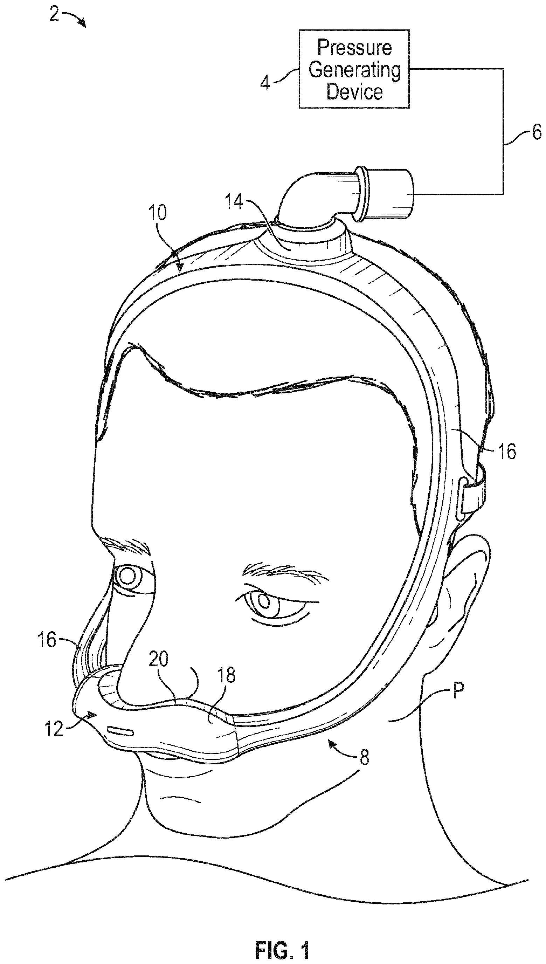

A respiratory interface system 2 adapted to provide a regimen of respiratory therapy to a patient P according to one exemplary embodiment of the present invention is shown in FIG. 1. Respiratory interface system 2 includes a pressure generating device 4 (shown schematically), and a delivery conduit 6 (shown schematically) fluidly coupled to a patient interface device 8. Pressure generating device 4 is structured to generate a flow of positive pressure breathing gas and may include, without limitation, ventilators, constant pressure support devices (such as a continuous positive airway pressure device, or CPAP device), variable pressure devices (e.g., BiPAP^{®}, Bi-Flex^{®}, or C-Flex^{™} devices manufactured and distributed by Philips Respironics of Murrysville, PA), and auto-titration pressure support devices. Delivery conduit 6 is structured to communicate the flow of breathing gas from pressure generating device 4 to patient interface device 8, and patient interface device 8 is structured to further communicate the flow of breathing gas received from conduit 6 to an airway of patient P. Delivery conduit 6 and patient interface device 8 are often collectively referred to as a patient circuit.

Continuing to refer to FIG. 1, patient interface device 8 includes a tubing assembly 10 and a mask 12 fluidly coupled to tubing assembly 10, both of which are sourced from the same manufacturer. Tubing assembly 10 includes a manifold portion 14 structured to receive the flow of positive pressure breathing gas from delivery conduit 6. In the example shown in FIG. 1, manifold portion 14 is positioned so as to be disposed generally at the top of the head of patient P, however, it is to be appreciated that manifold portion P may be positioned elsewhere without varying from the scope of the present invention. Tubing assembly 10 further includes a number (two are shown in the example of FIG. 1) of tubular portions 16 which each extend from manifold portion 14 to a distal end (not numbered) which is selectively coupled to mask 12. Each tubular portion 16 is structured to communicate the flow of positive pressure breathing gas from manifold portion 14 to the distal end thereof and onward to mask 12.

In the example shown in FIG. 1, each tubular portion 16 is sized and configured to extend down along a respective side of the head of patient P from manifold portion 14, and generally bend so as to curve along a respective cheek of the patient near the cheekbone of the patient, however, it is to be appreciated that each tubular portion 16 may extend along one or more other pathways without varying from the scope of the present invention. It is also to be appreciated that other arrangements aside from tubing assembly 10 may be employed in patient interface device 8 to provide the flow of positive pressure breathing gas produced by pressure generating device 4 to mask 12 without varying from the scope of the present invention.

Mask 12 includes a body 18 that defines a cavity (not numbered) therein that is structured to receive the flow of positive pressure breathing gas from one or more of the number of tubular portions 16 of tubing assembly 10. In the one example shown in FIG. 1, body 18 is made of a soft, flexible material, such as, without limitation, silicone, an appropriately soft thermoplastic elastomer, a closed-cell foam, or any other suitable material or combination of such materials, however, it is to be appreciated that body 18 may be formed from one or more other suitable materials without varying from the scope of the present invention.

Mask 12 further includes a number of apertures (not numbered) defined therein that is/are positioned and structured to communicate the flow of breathing gas from the cavity to the airway of the patient and a sealing element 20 disposed thereabout the number of apertures which is structured to sealingly engage about one or more of the nares and/or mouth of patient P. In the example embodiment illustrated in FIG. 1, sealing element 20 is formed as an integral portion of body 18, and thus is made of a soft, flexible material, such as, without limitation, silicone, an appropriately soft thermoplastic elastomer, a closed-cell foam, or any other suitable material or combination of such materials. It is to be appreciated, however, that sealing element 20 made be formed as a separate element and may take the form of any type of patient sealing element, such as a nasal/oral mask, a nasal pillow or a full face mask, which facilitates the delivery of the flow of breathing gas to the airway of a patient, without varying from the scope of the present invention.

Embodiments of the present invention provide for components of more than one manufacturer to be utilized together to form a patient interface device for use in a respiratory interface system such as previously described in conjunction with FIG. 1.

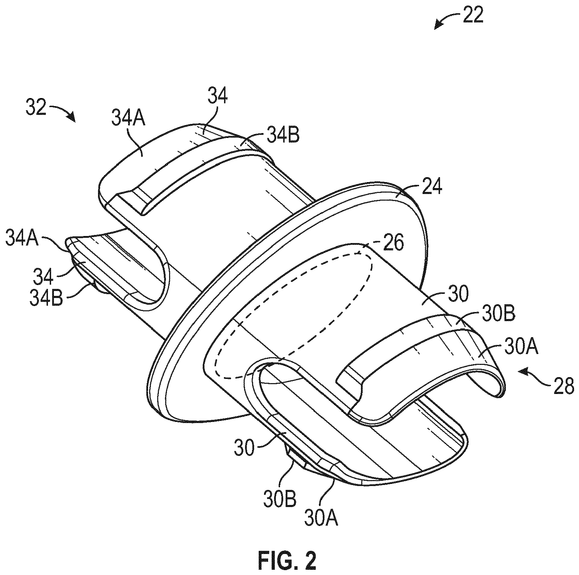

Referring now to FIG. 2, an adaptor 22 in accordance with one example embodiment of the present for use in coupling components of a patient interface device together is shown. Adaptor 22 is formed from a rigid or semi-rigid material or materials and includes a central flange portion 24 that extends generally radially outward from a central aperture 26 (shown in hidden line). Adaptor 22 further includes a first hollow male connector 28, which in the example shown in FIG. 2 is a pair of prongs 30 that extend outward from flange portion 24 in a first direction, and a second hollow male connector 32, which in the example shown in FIG. 2 is a second pair of prongs 34 that extend outward from flange portion 24 in a second direction opposite the first direction. Each of prongs 30 and 34 are arcuate shaped, in cross-section parallel to central flange portion 24, and include a tapered portion 30A, 34A at a leading end thereof that transitions to a raised portion 30B, 34B positioned inward (i.e., toward flange portion 24) from tapered portion 30A, 34A.

Each of first and second pairs of prongs 30 and 34 are structured to be inserted into, and cooperatively engage a correspondingly shaped aperture of one or both of a tubular portion or mask such as described in conjunction with FIG. 1. While first and second pairs of prongs 30 and 34 are shown as being of generally the same size and shape in the one example embodiment illustrated in FIG. 2, it is to be appreciated that first and second pairs of prongs 30 and 34 may be of different sizes and/or shapes depending on the corresponding shapes of the components they are intended to engage without varying from the scope of the present invention.

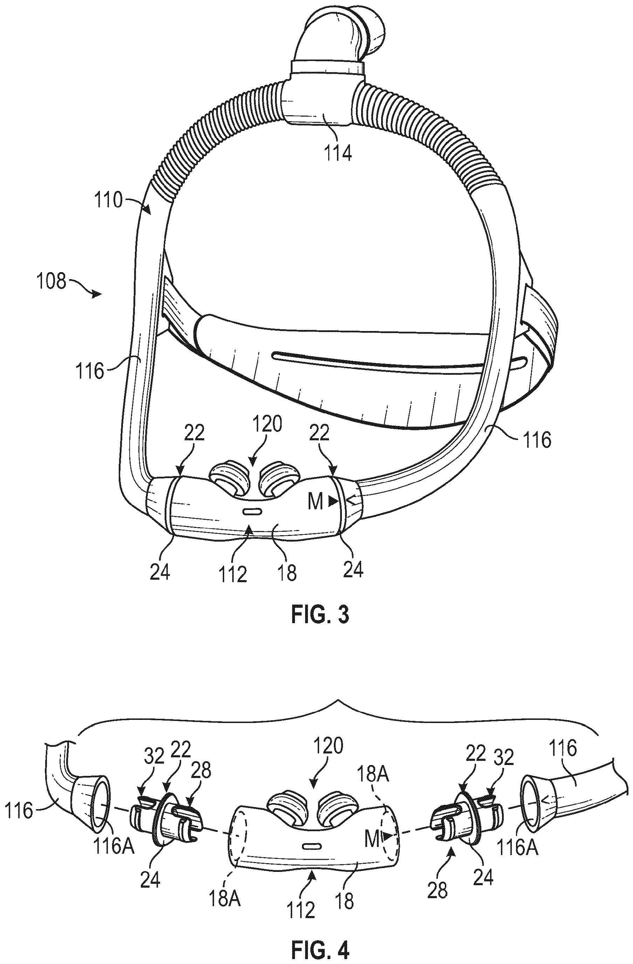

Referring now to FIGS. 3 and 4, a patient interface device 108 in accordance with one example embodiment of the present invention that may be employed with pressure generating device 4 in a respiratory interface system is shown. Patient interface device 108 is of a similar arrangement as patient interface device 8 previously discussed and as such includes a tubing assembly 110 and a mask 112. However, unlike patient interface device 8 in which tubing assembly 10 and mask 12 were from the same manufacturer and thus readily directly coupled together, tubing assembly 110 and mask 112 were produced by different manufacturers and thus not capable of being directly coupled. Hence, patient interface device 108 utilizes adaptor 22, and more particularly two of such adaptor 22, to couple tubing assembly 10 and mask 112 together to form patient interface 108.

Continuing to refer to FIGS. 3 and 4, tubing assembly 110 is similar to tubing assembly 10 and as such includes a manifold portion 114 structured to receive a flow of positive pressure breathing gas from pressure generating device 4 (e.g., via delivery conduit 6). Tubing assembly 110 further includes a number (two are shown in the example of FIG. 3) of tubular portions 116 which each extend from manifold portion 114 to a distal end (not numbered) which is selectively coupled to mask 112 via adaptor 22. Each tubular portion 116 is structured to communicate the flow of positive pressure breathing gas from manifold portion 114 to an aperture 116A (FIG. 4) defined in a distal end thereof. It is to be appreciated that each aperture 116A may generally be any suitable female coupling arrangement without varying from the scope of the present invention.

Mask 112 includes a body 18 that defines a cavity (not numbered) therein for receiving the flow of positive pressure breathing gas from one or more of the number of tubular portions 116 of tubing assembly 110. Mask 112 further includes a sealing element 120 in the form of a pair of nasal pillows that are each structured to sealingly engage with, and communicate the flow of breathing gas from the cavity to a respective nostril of a patient, however, it is to be appreciated that sealing element 120 may be any suitable arrangement, e.g., a nasal/oral interface, a nasal pillow or a full face interface, which facilitates the delivery of the flow of breathing gas from the cavity to the airway of a patient, without varying from the scope of the present invention. Mask 112 further includes a pair of apertures 18A (shown in dashed line in FIG. 4) defined in opposite ends of body 18 which are each structured to receive the flow of positive pressure breathing gas conveyed by tubing assembly 110 via adaptor 22. It is to be appreciated that each aperture 18A may generally be any suitable female coupling arrangement without varying from the scope of the present invention.

As previously indicated, mask 112 is coupled to tubing assembly 110 via two of adaptors 22. More particularly, in the one example shown in FIGS. 3 and 4 such coupling is accomplished by inserting first hollow male connector 28 (e.g., pair of prongs 30) into a female connector (e.g., aperture 18A) of mask 112 and second hollow male connector 32 (e.g., pair of prongs 34) into another female connector (e.g., aperture 116A) of the corresponding tubular portion 116 of tubing assembly 110. Such insertions may be carried out in any order or simultaneously. Tapered portions 30A and 34A of prongs 30 and 34 assist in first and second hollow male connectors 28 and 32 into the respective female connectors (e.g., apertures 18A and 116A), and raised portions 30B and 34B provide a click and/or snap-fit by interacting with correspondingly shaped portions of body 18 and tubular portion 116 once first and second hollow male connectors 28 and 32 have been sufficiently inserted into the corresponding female connectors. Such connections may readily be uncoupled by pulling in a direction opposite to the assembly direction. Such coupling of both sides is carried out in the same manner, and may be carried out in any order or simultaneously without varying from the scope of the present invention. It is to be appreciated that hollow male connectors 28 and 32 of adaptor 22 may be of any suitable arrangement for cooperatively engaging with the female connectors (i.e., apertures 116A and 18A) of tubing assembly 110 and mask 112.

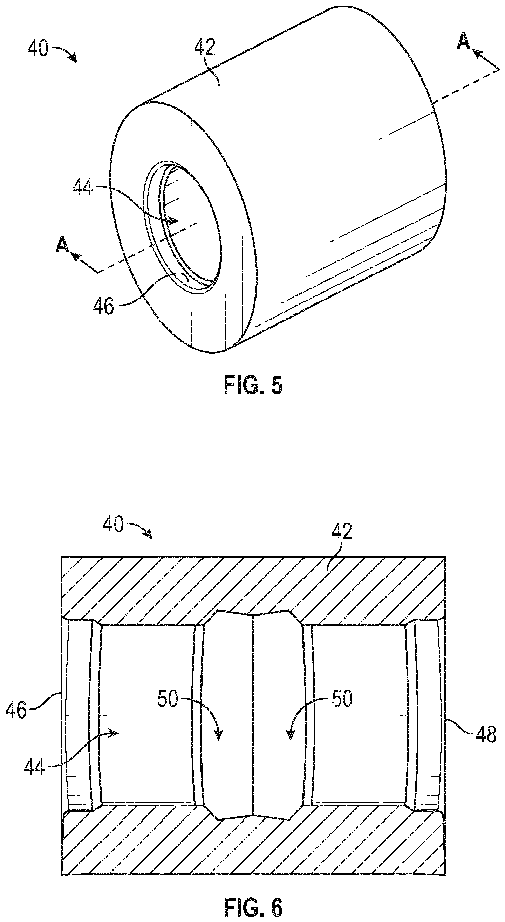

Referring now to FIGS. 5 and 6, another adaptor 40 in accordance with one example embodiment of the present for use in coupling components of a patient interface device together is shown. Adaptor 40 includes a body 42 formed from a rigid or semi-rigid material or materials (e.g., without limitation, plastic, silicone) that defines a passage 44 therethrough that extends between a first opening 46 and a second opening 48 defined in an outer surface (not numbered) of body 42. In the example shown in FIGS. 5 and 6, passage 44 has a generally elliptical cross-section, however, it is to be appreciated that passage 44 may be of other cross-sectional shape without varying from the scope of the present invention. Referring to the sectional view of FIG. 6, passage 44 may have a number of circumferential ridges or grooves 50 formed therein that extend part way, or completely around passage 44. Such ridges or grooves 50 are sized and configured to be engaged by cooperatively shaped portions of a connecting element inserted into passage 44, such as described below. It is to be appreciated that adaptor 40 generally defines two female connectors that are fluidly connected, and that other suitable female connectors of one or more of different size/style/shape beyond those illustrated herein may be employed without depending on the corresponding shapes of the components they are intended to engage without varying from the scope of the present invention.

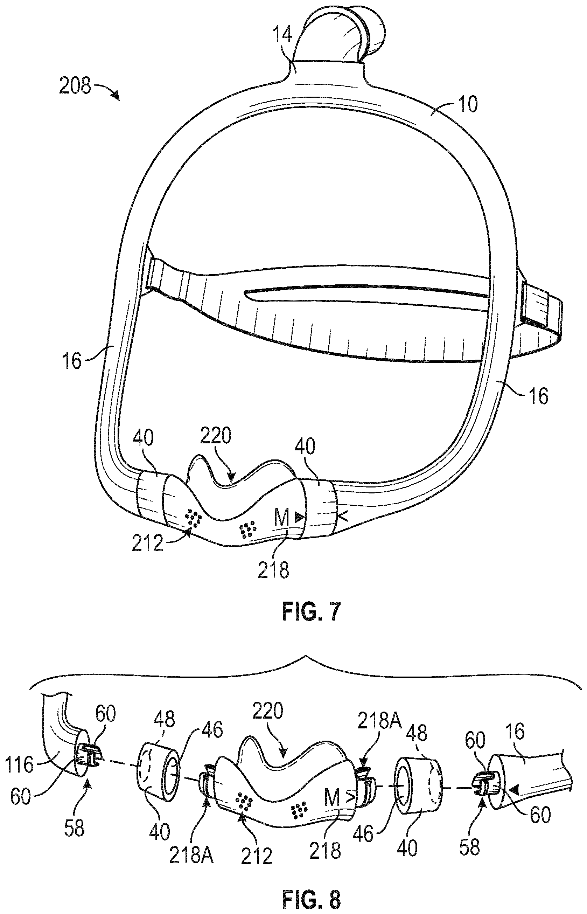

Referring now to FIGS. 7 and 8, a patient interface device 208 in accordance with one example embodiment of the present invention that may be employed with pressure generating device 4 in a respiratory interface system is shown. Patient interface device 208 is of a similar arrangement as patient interface device 8 previously discussed and as such includes tubing assembly 10, such as previously discussed in regard to FIG. 1, and a mask 212. However, unlike patient interface device 8 in which tubing assembly 10 and mask 12 were from the same manufacturer and thus readily directly coupled together, tubing assembly 10 and mask 212 were produced by different manufacturers and thus not capable of being directly coupled. Hence, patient interface device 208 utilizes adaptor 40, and more particularly two of such adaptor 40, to couple tubing assembly 10 and mask 212 together to form patient interface 208.

As previously discussed in regard to FIG. 1, tubing assembly 10 includes a manifold portion 14 structured to receive a flow of positive pressure breathing gas from pressure generating device 4 (e.g., via delivery conduit 6), and a number (two are shown in the example of FIG. 7) of tubular portions 16 which each extend from manifold portion 14 to a distal end (not numbered) which is selectively coupled to mask 212 via adaptor 40. Each tubular portion 16 is structured to communicate the flow of positive pressure breathing gas from manifold portion 14 to a hollow male connector 58, which in the example shown in FIG. 8 is a pair of prongs 60 that extend outward from the distal end of tubular portion 16. In the example shown in FIG. 8, each hollow male connector 58 is of a similar arrangement as hollow male connectors 28 and 32 of adaptor 22, however, it is to be appreciated that each hollow male connector 58 may be of any other suitable arrangement without varying from the scope of the present invention.

Mask 212 includes a body 218 that defines a cavity (not numbered) therein for receiving the flow of positive pressure breathing gas from one or more of the number of tubular portions 16 of tubing assembly 10. Mask 212 further includes a sealing element 220 in the form of a nasal pillow that is structured to sealingly engage with, and communicate the flow of breathing gas from the cavity to the nostrils of a patient, however, it is to be appreciated that sealing element 220 may be any suitable arrangement, e.g., a nasal/oral interface, a nasal pillow/pillows or a full face interface, which facilitates the delivery of the flow of breathing gas to the airway of a patient, without varying from the scope of the present invention. Mask 212 further includes a pair of hollow male connectors 218A extending from opposite ends of body 218 that are each structured to receive the flow of positive pressure breathing gas conveyed by tubular portion 16 of tubing assembly 10 via adaptor 40. In the example shown in FIG. 8, each hollow male connector 218A is of a similar arrangement as hollow male connectors 28 and 32 of adaptor 22, as well as hollow male connector 58 of tubing assembly 10, however, it is to be appreciated that each hollow male connector 218A may be of any other suitable arrangement without varying from the scope of the present invention.

As previously indicated, mask 212 is coupled to tubing assembly 10 via two of such adaptor 40. More particularly, in the one example shown in FIGS. 7 and 8 such coupling is accomplished by inserting hollow male connector 58 of one tubular portion 16 into the corresponding female connector (e.g., aperture 48) of adaptor 40 and inserting the corresponding hollow male connector 218A of mask 210 into the other female connector (e.g., aperture 46) of adaptor 40 (in any order or simultaneously). Such process is repeated for the other adaptor 40. The connections formed via adaptor 40 may readily be uncoupled by pulling in a direction opposite to the assembly direction. Such coupling of both sides is carried out in the same manner, and may be carried out in any order or simultaneously without varying from the scope of the present invention.

From the foregoing examples, it is thus to be appreciated that adaptors in accordance with the present invention provide for components of patient interface devices that would otherwise not be connectable to be readily connected. As a result of such new connectability, patient interface devices can readily be created thus providing patients with greater options for devices to be used in treating their medical conditions.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A respiratory interface system (2) for use in delivering a flow of a positive pressure breathing gas to an airway of a patient, the respiratory interface system comprising a patient interface device (8) comprising:
a tubing assembly (10) structured to be disposed on the head of the patient;
a mask (12) having a sealing element (20) structured to sealingly engage about the airway of the patient; and
an adaptor (22, 40), wherein the mask is coupled to the tubing assembly via the adaptor, and wherein the tubing assembly, the mask, and the adaptor define a pathway structured to conduct the flow of the positive pressure breathing gas to the airway of the patient, and
**characterized in that** the adaptor comprises:
a first hollow male connector (28) and a second hollow male connector (32) for insertion respectively in correspondingly-shaped female connectors (18A, 116A) of the tubing assembly and the mask; or
a first female connector (46) and a second female connector (48) for receipt respectively of correspondingly-shaped male connectors (58, 218A) of the tubing assembly and the mask.

2. The respiratory interface system of claim 1, wherein the tubing assembly comprises:
a manifold portion (14) structured to be disposed at or about the top of the head of the patient; and
a number of tubular portions (16), each extending from the manifold portion to a distal end that is coupled to the mask via the adaptor.

3. The respiratory interface system of claim 2, wherein each tubular portion is sized and configured to extend down along a respective side of the head of the patient from the manifold portion and generally bend so as to curve along a respective cheek of the patient near the cheekbone of the patient.

4. The respiratory interface system of claim 1:
wherein the adaptor (22) comprises a central flange portion (24) that extends generally radially outward from a central aperture;
wherein the first hollow male connector of the adaptor extends from the central flange and is coupled with a correspondingly-shaped female connector of the tubing assembly; and
wherein the second male connector of the adaptor extends from the central flange and is coupled with a correspondingly-shaped female connector of the mask.

5. The respiratory interface system of claim 4, wherein the first hollow male connector comprises a first pair of prongs (30) that extend outward from the flange portion in a first direction, and wherein the second hollow male connector comprises a second pair of prongs (34) that extend outward from the flange portion in a second direction opposite the first direction.

6. The respiratory interface system of claim 5, wherein each prong of the first pair of prongs and the second pair of prongs is arcuate shaped in a cross-section parallel to the central flange portion, and wherein each prong comprises a tapered portion (30A, 34A) at a leading end thereof that transitions to a raised portion (30B, 34B) positioned inward toward the flange portion from the tapered portion.

7. The respiratory interface system of claim 1, wherein the adaptor (40) comprises: a body (42) defining a passage (44) therethrough that extends between a first opening and a second opening defined in an outer surface of the body, wherein the first opening defines the first female connector coupled with a correspondingly-shaped hollow male connector of the mask, and wherein the second opening defines the second female connector coupled with a correspondingly-shaped hollow male connector (58) of the tubing assembly.

8. The respiratory interface system of claim 7, wherein the hollow male connector of the mask comprises a first pair of prongs that extend outward from an end of the mask, and wherein the hollow male connector of the tubing assembly comprises a second pair of prongs (60) that extend outward from a distal end of a tubular portion of the tubing assembly.

9. The respiratory interface system of claim 8, wherein each prong of the first pair of prongs and the second pair of prongs is arcuate shaped in a cross-section, and wherein each prong comprises a tapered portion at a leading end thereof that transitions to a raised portion positioned inward from the tapered portion.

10. The respiratory interface system of claim 1, further comprising:
a pressure generating device (4) structured to produce the flow of the positive pressure breathing gas; and
a delivery conduit (6) fluidly coupled between the pressure generating device and the tubing assembly, the delivery conduit structured to convey the flow of the positive pressure breathing gas from the pressure generating device to the tubing assembly.

11. A method of assembling a patient interface device (8) for use in providing a flow of a positive pressure breathing gas to an airway of a patient, the patient interface device including: a mask (12) having a sealing element (20) structured to sealingly engage about the airway of the patient, and a tubing assembly (10) having a manifold portion (14) and a pair of tubular portions (16), each tubular portion extending from the manifold portion to a distal end, wherein the tubing assembly is structured to convey the flow of the positive pressure breathing gas to the mask and the mask is further structured to convey the flow to the airway of the patient, wherein the patient interface device comprises a first adaptor (22, 40) and a second adaptor (22, 40),
wherein each of the first and second adaptors comprises:
a first hollow male connector (28) and a second hollow male connector (32) for insertion respectively in correspondingly-shaped female connectors (18A, 116A) of the tubing assembly and the mask; or
a first female connector (46) and a second female connector (48) for receipt respectively of correspondingly-shaped male connectors (58, 218A) of the tubing assembly and the mask,
the method comprising:
connecting the distal end of a first one of the pair of tubular portions to the mask via the first adaptor; and
connecting the distal end of a second one of the pair of tubular portions to the mask via the second adaptor.

12. The method of claim 11:
wherein connecting the distal end of the first one of the pair of tubular portions to the mask via the first adaptor comprises:
inserting the first hollow male connector of the first adaptor into a female connector (18A) of the mask; and
inserting the second hollow male connector of the first adaptor into a female connector (116A) of the first one of the pair of tubular portions, and
wherein connecting the distal end of the second one of the pair of tubular portions to the mask via a second adaptor comprises:
inserting the first hollow male connector of the second adaptor into a female connector (18A) of the mask; and
inserting the second hollow male connector of the second adaptor into a female connector (116A) of the second one of the pair of tubular portions.

13. The method of claim 11:
wherein connecting the distal end of the first one of the pair of tubular portions to the mask via the first adaptor comprises:
inserting a hollow male connector (58) of the first one of the pair of tubular portions into the first female connector of the first adaptor; and
inserting a first hollow male connector (218A) of the mask into the second female connector of the first adaptor, and
wherein connecting the distal end of the second one of the pair of tubular portions to the mask via a second adaptor comprises:
inserting a hollow male connector (58) of the second one of the pair of tubular portions into the first female connector of the second adaptor; and
inserting a second hollow male connector (218A) of the mask into the second female connector of the second adaptor.

## Patentansprüche

1. Atmungsschnittstellensystem (2) zur Verwendung bei der Abgabe eines Stroms eines Überdruck-Atemgases an die Atemwege eines Patienten, wobei das Atmungsschnittstellensystem eine Patientenschnittstellenvorrichtung (8) umfasst, die Folgendes umfasst:
eine Schlauchanordnung (10), die dazu strukturiert ist, an dem Kopf des Patienten angeordnet zu sein;
eine Maske (12), die ein Dichtungselement (20) aufweist, das dazu strukturiert ist, abdichtend um die Atemwege des Patienten einzugreifen; und
einen Adapter (22, 40), wobei die Maske über den Adapter mit der Schlauchanordnung gekoppelt ist und wobei die Schlauchanordnung, die Maske und der Adapter einen Weg definieren, der derart strukturiert ist, dass er den Strom des Überdruckatemgases zu den Atemwegen des Patienten leitet, und
**dadurch gekennzeichnet, dass** der Adapter Folgendes umfasst:
einen ersten hohlen Steckverbinder (28) und einen zweiten hohlen Steckverbinder (32) zum Einführen in entsprechend geformte Steckbuchsen (18A, 116A) der Schlauchanordnung und der Maske; oder
eine ersten Steckbuchse (46) und eine zweite Steckbuchse (48) zum Aufnehmen jeweils entsprechend geformter Steckverbindern (58, 218A) der Schlauchanordnung bzw. der Maske.

2. Atmungsschnittstellensystem nach Anspruch 1, wobei die Schlauchanordnung Folgendes umfasst:
einen Verteilerabschnitt (14), der dazu strukturiert ist, an der oder um die Oberseite des Kopfes des Patienten angeordnet zu sein; und
eine Anzahl röhrenförmiger Abschnitte (16), die sich jeweils von dem Verteilerabschnitt zu einem distalen Ende erstrecken, das über den Adapter mit der Maske gekoppelt ist.

3. Atmungschnittstellensystem nach Anspruch 2, wobei jeder röhrenförmige Abschnitt dazu bemessen und konfiguriert ist, sich von dem Verteilerabschnitt entlang einer jeweiligen Seite des Kopfes des Patienten nach unten zu erstrecken und sich im Allgemeinen derart zu biegen, dass er entlang einer jeweiligen Wange des Patienten nahe dem Wangenknochen des Patienten verläuft.

4. Atmungschnittstellensystem nach Anspruch 1:
wobei der Adapter (22) einen zentralen Flanschabschnitt (24) umfasst, der sich im Allgemeinen radial von einer zentralen Öffnung nach außen erstreckt;
wobei sich der erste hohle Steckverbinder des Adapters von dem zentralen Flansch erstreckt und mit einer entsprechend geformten Steckbuchse der Schlauchanordnung gekoppelt ist; und
wobei sich der zweite Steckverbinder des Adapters von dem Mittelflansch erstreckt und mit einer entsprechend geformten Steckbuchse der Maske gekoppelt ist.

5. Atmungschnittstellensystem nach Anspruch 4, wobei der erste hohle Steckverbinder ein erstes Paar Zinken (30) umfasst, die sich von dem Flanschabschnitt in einer ersten Richtung nach außen erstrecken, und wobei der zweite hohle Steckverbinder ein zweites Paar Zinken (34) umfasst, die sich von dem Flanschabschnitt in einer zweiten Richtung, die der ersten Richtung entgegengesetzt ist, nach außen erstrecken.

6. Atmungschnittstellensystem nach Anspruch 5, wobei jede Zinke des ersten Paares Zinken und des zweiten Paares Zinken in einem Querschnitt parallel zu dem zentralen Flanschabschnitt bogenförmig geformt ist, und wobei jede Zinke an einem vorderen Ende davon einen verjüngten Abschnitt (30A, 34A) umfasst, der in einen erhabenen Abschnitt (30B, 34B) übergeht, der von dem verjüngten Abschnitt nach innen in Richtung des Flanschabschnitts positioniert ist.

7. Atmungschnittstellensystem nach Anspruch 1, wobei der Adapter (40) Folgendes umfasst: einen Körper (42), der einen Durchgang (44) definiert, der sich zwischen einer ersten Öffnung und einer zweiten Öffnung erstreckt, die in einer Außenfläche des Körpers definiert sind, wobei die erste Öffnung die ersten Steckbuchse definiert, die mit einem entsprechend geformten hohlen Steckverbinder der Maske gekoppelt ist, und wobei die zweite Öffnung die zweite Steckbuchse definiert, die mit einem entsprechend geformten hohlen Steckverbinder (58) der Schlauchanordnung gekoppelt ist.

8. Atmungschnittstellensystem nach Anspruch 7, wobei der hohle Steckverbinder der Maske ein erstes Paar Zinken umfasst, die sich von einem Ende der Maske nach außen erstrecken, und wobei der hohle Steckverbinder der Schlauchanordnung ein zweites Paar Zinken (60) umfasst, die sich von einem distalen Ende eines röhrenförmigen Abschnitts der Schlauchanordnung nach außen erstrecken.

9. Atmungschnittstellensystem nach Anspruch 8, wobei jede Zinke des ersten Paares Zinken und des zweiten Paares Zinken im Querschnitt bogenförmig ist, und wobei jede Zinke an ihrem vorderen Ende einen verjüngten Abschnitt aufweist, der in einen erhabenen Abschnitt übergeht, der von dem verjüngten Abschnitt nach innen positioniert ist.

10. Atmungschnittstellensystem nach Anspruch 1, weiter umfassend:
eine Druckerzeugungsvorrichtung (4), die derart aufgebaut ist, dass sie den Strom des Überdruck-Atemgases erzeugt; und
eine Zufuhrleitung (6), die fluidisch zwischen der Druckerzeugungsvorrichtung und der Schlauchanordnung gekoppelt ist, wobei die Zufuhrleitung dazu strukturiert ist, den Strom des Überdruck-Atemgases von der Druckerzeugungsvorrichtung zu der Schlauchanordnung zu leiten.

11. Verfahren zum Zusammenbauen einer Patientenschnittstellenvorrichtung (8) zur Verwendung beim Bereitstellen eines Stroms von Überdruck-Atemgas zu den Atemwegen eines Patienten, wobei die Patientenschnittstellenvorrichtung beinhaltet: eine Maske (12), die ein Dichtungselement (20) aufweist, das derart strukturiert ist, dass es abdichtend um die Atemwege des Patienten eingreift, und eine Schlauchanordnung (10), die einen Verteilerabschnitt (14) und ein Paar röhrenförmiger Abschnitte (16) aufweist, wobei sich jeder röhrenförmige Abschnitt von dem Verteilerabschnitt zu einem distalen Ende erstreckt, wobei die Schlauchanordnung dazu strukturiert ist, den Strom des Überdruck-Atemgases zu der Maske zu leiten, und die Maske weiter dazu strukturiert ist, den Strom zu den Atemwegen des Patienten zu leiten, wobei die Patientenschnittstellenvorrichtung einen ersten Adapter (22, 40) und einen zweiten Adapter (22, 40) umfasst,
wobei der erste und der zweite Adapter jeweils Folgendes umfassen:
einen ersten hohlen Steckverbinder (28) und einen zweiten hohlen Steckverbinder (32) zum Einführen in entsprechend geformte Steckbuchsen (18A, 116A) der Schlauchanordnung und der Maske; oder
eine erste Steckbuchse (46) und eine zweite Steckbuchse (48) zum Aufnehmen von entsprechend geformten Steckverbindern (58, 218A) der Schlauchanordnung bzw. der Maske,
wobei das Verfahren Folgendes umfasst:
Verbinden des distalen Endes eines ersten des Paares röhrenförmiger Abschnitte mit der Maske über den ersten Adapter; und
Verbinden des distalen Endes eines zweiten des Paares röhrenförmiger Abschnitte mit der Maske über den zweiten Adapter.

12. Verfahren nach Anspruch 11:
wobei das Verbinden des distalen Endes des ersten des Paares röhrenförmiger Abschnitte mit der Maske über den ersten Adapter Folgendes umfasst:
Einführen des ersten hohlen Steckverbinders des ersten Adapters in eine Steckbuchse (18A) der Maske; und
Einführen des zweiten hohlen Steckverbinders des ersten Adapters in eine Steckbuchse (116A) des ersten des Paares röhrenförmiger Abschnitte, und
wobei das Verbinden des distalen Endes des zweiten des Paares röhrenförmiger Abschnitte mit der Maske über einen zweiten Adapter Folgendes umfasst:
Einführen des ersten hohlen Steckverbinders des zweiten Adapters in eine Steckbuchse (18A) der Maske; und
Einführen des zweiten hohlen Steckverbinders des zweiten Adapters in eine Steckbuchse (116A) des zweiten Teils des Paares röhrenförmiger Abschnitte.

13. Verfahren nach Anspruch 11:
wobei das Verbinden des distalen Endes des ersten des Paares röhrenförmiger Abschnitte mit der Maske über den ersten Adapter Folgendes umfasst:
Einführen eines hohlen Steckverbinders (58) des ersten des Paares röhrenförmiger Abschnitte in die erste Steckbuchse des ersten Adapters; und
Einführen eines ersten hohlen Steckverbinders (218A) der Maske in die zweite Steckbuchse des ersten Adapters, und
wobei das Verbinden des distalen Endes des zweiten des Paares röhrenförmiger Abschnitte mit der Maske über einen zweiten Adapter Folgendes umfasst:
Einführen eines hohlen Steckverbinders (58) des zweiten Teils des Paares röhrenförmiger Teile in die erste Steckbuchse des zweiten Adapters; und
Einführen eines zweiten hohlen Steckverbinders (218A) der Maske in die zweite Steckbuchse des zweiten Adapters.

## Revendications

1. Système d'interface respiratoire (2) destiné à être utilisé pour délivrer un flux d'un gaz respiratoire à pression positive vers les voies respiratoires d'un patient, le système d'interface respiratoire comprenant un dispositif d'interface patient (8) comprenant :
un ensemble tube (10) structuré pour être disposé sur la tête du patient ;
un masque (12) présentant un élément d'étanchéité (20) structuré pour s'engager de manière étanche autour des voies respiratoires du patient ; et
un adaptateur (22, 40), dans lequel le masque est couplé à l'ensemble tube via l'adaptateur, et dans lequel l'ensemble tube, le masque et l'adaptateur définissent un trajet structuré pour conduire le flux du gaz respiratoire à pression positive vers les voies respiratoires du patient, et
**caractérisé en ce que** l'adaptateur comprend :
un premier connecteur mâle creux (28) et un second connecteur mâle creux (32) destinés à être insérés respectivement dans des connecteurs femelles de forme correspondante (18A, 116A) de l'ensemble tube et du masque ; ou
un premier connecteur femelle (46) et un second connecteur femelle (48) pour recevoir respectivement des connecteurs mâles de forme correspondante (58, 218A) de l'ensemble tube et du masque.

2. Système d'interface respiratoire selon la revendication 1, dans lequel l'ensemble tube comprend :
une partie collecteur (14) structurée pour être disposée au niveau ou sur le sommet de la tête du patient ; et
un nombre de parties tubulaires (16), chacune s'étendant de la partie collecteur jusqu'à une extrémité distale qui est couplée au masque via l'adaptateur.

3. Système d'interface respiratoire selon la revendication 2, dans lequel chaque partie tubulaire est dimensionnée et configurée pour s'étendre vers le bas le long d'un côté respectif de la tête du patient à partir de la partie collecteur et se plier généralement de manière à s'incurver le long d'une joue respective du patient près de la pommette du patient.

4. Système d'interface respiratoire selon la revendication 1 :
dans lequel l'adaptateur (22) comprend une partie de bride centrale (24) qui s'étend généralement radialement vers l'extérieur à partir d'une ouverture centrale ;
dans lequel le premier connecteur mâle creux de l'adaptateur s'étend à partir de la bride centrale et est couplé à un connecteur femelle de forme correspondante de l'ensemble tube ; et
dans lequel le second connecteur mâle de l'adaptateur s'étend à partir de la bride centrale et est couplé à un connecteur femelle de forme correspondante du masque.

5. Système d'interface respiratoire selon la revendication 4, dans lequel le premier connecteur mâle creux comprend une première paire de broches (30) qui s'étendent vers l'extérieur depuis la partie de bride dans une première direction, et dans lequel le second connecteur mâle creux comprend une seconde paire de broches (34) qui s'étendent vers l'extérieur à partir de la partie de bride dans une seconde direction opposée à la première direction.

6. Système d'interface respiratoire selon la revendication 5, dans lequel chaque branche de la première paire de branches et de la seconde paire de branches est de forme arquée dans une section transversale parallèle à la partie de bride centrale, et dans lequel chaque branche comprend une partie effilée (30A, 34A) au niveau d'une extrémité avant de celle-ci qui passe en une partie surélevée (30B, 34B) positionnée vers l'intérieur vers la partie de bride à partir de la partie effilée.

7. Système d'interface respiratoire selon la revendication 1, dans lequel l'adaptateur (40) comprend : un corps (42) définissant un passage (44) à travers celui-ci qui s'étend entre une première ouverture et une seconde ouverture définies dans une surface externe du corps, dans lequel la première ouverture définit le premier connecteur femelle couplé à un connecteur mâle creux de forme correspondante du masque, et dans lequel la seconde ouverture définit le second connecteur femelle couplé à un connecteur mâle creux de forme correspondante (58) de l'ensemble tube.

8. Système d'interface respiratoire selon la revendication 7, dans lequel le connecteur mâle creux du masque comprend une première paire de broches qui s'étendent vers l'extérieur à partir d'une extrémité du masque, et dans lequel le connecteur mâle creux de l'ensemble tube comprend une seconde paire de broches (60) qui s'étendent vers l'extérieur à partir d'une extrémité distale d'une partie tubulaire de l'ensemble tube.

9. Système d'interface respiratoire selon la revendication 8, dans lequel chaque branche de la première paire de branches et de la seconde paire de branches est de forme arquée dans une section transversale, et dans lequel chaque branche comprend une partie effilée au niveau d'une extrémité avant de celle-ci qui passe en une partie surélevée positionnée vers l'intérieur à partir de la partie effilée.

10. Système d'interface respiratoire selon la revendication 1, comprenant en outre :
un dispositif de génération de pression (4) structuré pour produire le flux du gaz respiratoire à pression positive ; et
un conduit de délivrance (6) couplé fluidiquement entre le dispositif de génération de pression et l'ensemble tube, le conduit de délivrance étant structuré pour transporter le flux de gaz respiratoire à pression positive du dispositif de génération de pression vers l'ensemble tube.

11. Procédé d'assemblage d'un dispositif d'interface patient (8) destiné à être utilisé pour fournir un flux d'un gaz respiratoire à pression positive vers les voies respiratoires d'un patient, le dispositif d'interface patient incluant : un masque (12) présentant un élément d'étanchéité (20) structuré pour s'engager de manière étanche autour des voies respiratoires du patient, et un ensemble tube (10) présentant une partie collecteur (14) et une paire de parties tubulaires (16), chaque partie tubulaire s'étendant depuis la partie collecteur jusqu'à une extrémité distale, dans lequel l'ensemble tube est structuré pour acheminer le flux du gaz respiratoire à pression positive vers le masque et le masque est en outre structuré pour acheminer le flux vers les voies respiratoires du patient, dans lequel le dispositif d'interface patient comprend un premier adaptateur (22, 40) et un second adaptateur (22, 40),
dans lequel chacun des premier et second adaptateurs comprend :
un premier connecteur mâle creux (28) et un second connecteur mâle creux (32) destinés à être insérés respectivement dans des connecteurs femelles de forme correspondante (18A, 116A) de l'ensemble tube et du masque ; ou
un premier connecteur femelle (46) et un second connecteur femelle (48) destinés à recevoir respectivement des connecteurs mâles de forme correspondante (58, 218A) de l'ensemble tube et du masque,
le procédé comprenant les étapes consistant à :
connecter l'extrémité distale d'une première de la paire de parties tubulaires au masque via le premier adaptateur ; et
connecter l'extrémité distale d'une seconde de la paire de parties tubulaires au masque via le second adaptateur.

12. Procédé selon la revendication 11 :
dans lequel la connexion de l'extrémité distale de la première de la paire de parties tubulaires avec le masque via le premier adaptateur comprend les étapes consistant à :
insérer le premier connecteur mâle creux du premier adaptateur dans un connecteur femelle (18A) du masque ; et
insérer le second connecteur mâle creux du premier adaptateur dans un connecteur femelle (116A) de la première de la paire de parties tubulaires, et
dans lequel la connexion de l'extrémité distale de la seconde de la paire de parties tubulaires avec le masque via un second adaptateur comprend :
insérer le premier connecteur mâle creux du second adaptateur dans un connecteur femelle (18A) du masque ; et
insérer le second connecteur mâle creux du second adaptateur dans un connecteur femelle (116A) de la seconde de la paire de parties tubulaires.

13. Procédé selon la revendication 11 :
dans lequel la connexion de l'extrémité distale de la première de la paire de parties tubulaires avec le masque via le premier adaptateur comprend les étapes consistant à :
insérer un connecteur mâle creux (58) de la première de la paire de parties tubulaires dans le premier connecteur femelle du premier adaptateur ; et
insérer un premier connecteur mâle creux (218A) du masque dans le second connecteur femelle du premier adaptateur, et
dans lequel la connexion de l'extrémité distale de la seconde de la paire de parties tubulaires avec le masque via un second adaptateur comprend :
insérer un connecteur mâle creux (58) de la seconde de la paire de parties tubulaires dans le premier connecteur femelle du second adaptateur ; et
insérer un second connecteur mâle creux (218A) du masque dans le second connecteur femelle du second adaptateur.
